# EUROPEAN PATENT SPECIFICATION

(11) **EP 4 539 896 B1**
(45) Date of publication and mention of the grant of the patent: **05.08.2026**
(21) Application number: 23733711.8
(22) Date of filing: 15.06.2023
(51) Int. Cl.: A61L 9/20

(54) **LED FILAMENT COMPRISING LEDS ARRANGED TO EMIT VIOLET AND ULTRAVIOLET LIGHT**
LED-FADEN MIT LEDS, DIE VIOLETTES UND ULTRAVIOLETTES LICHT EMITTIEREN
FILAMENT À DEL COMPRENANT DES DEL AGENCÉES POUR ÉMETTRE DE LA LUMIÈRE VIOLETTE ET ULTRAVIOLETTE

(30) Priority: 20.06.2022 EP 22179790
(43) Date of publication of application: 23.04.2025
(73) Proprietor: Signify Holding B.V., 5656 AE Eindhoven (NL)
(72) Inventor: VAN BOMMEL, Ties, 5656 AE Eindhoven (NL)
(74) Representative: Verweij, Petronella Daniëlle
(86) International application number: PCT/EP2023/066118
(87) International publication number: WO 2023/247321

(56) References cited:
- WO-A1-2022/005505
- CN-A- 111 029 452
- CN-U- 203 842 065
- KR-B1- 102 404 462
- US-A1- 2019 234 563

## Description

### FIELD OF THE INVENTION

The present invention generally relates to light emitting diode, LED, filaments. More specifically, the present invention is related to LED filaments arranged to emit a combination of ultraviolet, UV, light and violet light.

### BACKGROUND OF THE INVENTION

The use of light emitting diodes (LED) for illumination purposes continues to attract attention. Compared to incandescent lamps, fluorescent lamps, neon tube lamps, etc., LEDs provide numerous advantages such as a longer operational life, a reduced power consumption, and an increased efficiency related to the ratio between light energy and heat energy. In particular, LED filament lamps are highly appreciated as they are very decorative.

Due to the advantageous aspects of the use of LEDs, the interest has rapidly increased to replace conventional light sources with LEDs in many lighting arrangements. It will be appreciated that this replacement, also called retrofitting, is appreciated and desired by users who wish to have the look of an incandescent bulb. The light source replacement (retrofitting) is often performed by removing the conventional light source(s) from the luminaire (e.g. a lamp holder) of the lighting arrangement and attaching the LEDs, LED arrangement(s) or LED device(s) into the luminaire. One of these concepts is based on LED filaments which are placed in a bulb, as the appearance of lamps of this kind are appreciated as they are highly decorative.

Furthermore, it is of interest to combine the advantageous properties of LED filaments with respect to aesthetics and light distribution purposes according to the above with the advantageous properties of disinfection (bactericidal) lighting. It will be appreciated that disinfection lighting has become a topic of renewed interest as the demand for sterilization increases. For example, UVA (315-400 nm) and/or violet light (400-420 nm) can be used for disinfection purposes, e.g. inactivating/killing bacteria.

It should also be noted that it is a wish to replace older technologies (e.g. mercury-based UV tubes) with UV LED-based solutions. Furthermore, existing arrangements comprising LEDs arranged to emit UV light may suffer from complexity and/or operational issues regarding efficiency characteristics and/or safety.

Hence, it is an object of the present invention to combine the advantageous properties of LEDs with respect to energy efficiency and light distribution purposes with the advantageous properties of disinfection (bactericidal and/or viricidal) lighting. It is also an object of the present invention to overcome at least some complexity and/or operational issues regarding current/voltage driving characteristics, efficiency characteristics and/or safety. Documents CN 203842065 U and KR 102404462 B1 show different LED-based sterilization devices.

### SUMMARY OF THE INVENTION

It is of interest to combine the advantageous properties of LEDs with respect energy efficiency, light distribution purposes and/or aesthetics with the advantageous properties of providing disinfection (bactericidal and/or viricidal) lighting, whilst providing an efficient and/or safely operated LED arrangement.

This and other objects are achieved by providing a LED filament having the features in the independent claim. Preferred embodiments are defined in the dependent claims.

Hence, according to the present invention, there is provided a light emitting diode, LED, filament, configured to emit LED filament light. The LED filament comprises a first linear array of a plurality of first light emitting diodes, LEDs, wherein the plurality of first LEDs is arranged to emit violet light in a wavelength range of 380-420 nm. The LED filament further comprises a second linear array of a plurality of second LEDs, wherein the plurality of second LEDs is arranged to emit ultra-violet, UV, light in a wavelength range of 100-380 nm, and wherein the second array is arranged adjacent and parallel to the first array. The LED filament further comprises a first encapsulant at least partially enclosing the first array of the plurality of first LEDs, wherein the first encapsulant comprises a light-scattering material configured to scatter the violet light emitted from the plurality of first LEDs.

Thus, the present invention is based on the idea of providing a LED filament comprising a first linear array of LEDs configured to emit violet light via a light-scattering encapsulant, and a second linear array of LEDs configured to emit primarily non-scattered ultra-violet light. The first and second linear array are arranged adjacent each other and parallel to each other, such that adjacent and parallel line emissions of violet and UV light, respectively, may be achieved. Hence, there is provided a LED filament which is able to efficiently and safely provide disinfective UV light whilst providing visible violet light.

The present invention is advantageous in that the linear arrays of the plurality of LEDs provide two adjacent and parallel line emissions of violet and UV light respectively, providing a combination of visible violet light and disinfecting UV light.

The present invention is further advantageous in that the visible violet light may be decorative and aesthetically pleasing, and simultaneously indicate when and where the UV light is being emitted. As UV light may be harmful to people, the violet light emitted by the plurality of first LEDs provides an increased safety.

The present invention is also advantageous in that the plurality of first LEDs enclosed by a light-scattering encapsulant may emit violet light in a scattered manner, providing more efficient and aesthetically pleasing visible light, while the second LEDs are arranged outside the light scattering encapsulant, such that no UV light is scattered back to the LED filament. By not scattering back the UV light, the risk of damaging the LED filament with UV light is significantly decreased, since the UV light may be harmful to components of the LED filament.

The present invention is further advantageous in that the linear arrays of the plurality of LEDs of the LED filament allow for a non-complex and convenient electric circuitry. In turn, this increases the service life of the LED filament and/or reduces the risk of malfunction thereof at operation.

The present invention is advantageous in that the LEDs are relatively small, and the LED filaments providing UV light may be smaller than conventional UV lights. As a consequence, the present UV LED filaments may be more convenient and versatile.

It will be appreciated that that the wavelength, 380-420 nm, of the first LEDs provides both visible light and at least partially disinfecting light.

It will be further appreciated that the second LEDs provide UV light with disinfecting properties.

It will be further appreciated that the LED filament of the present invention furthermore comprises relatively few components. The relatively low number of components is advantageous in that the LED filament is relatively inexpensive to fabricate. Moreover, the relatively low number of components of the LED filament implies an easier recycling, especially compared to devices or arrangements comprising a relatively high number of components which impede an easy disassembling and/or recycling operation.

The LED filament, which is configured to emit LED filament light comprises a first linear array of a plurality of first LEDs, wherein the plurality of first LEDs is arranged to emit violet light in a wavelength range of 380-420 nm. The LED filament further comprises a second linear array of a plurality of second LEDs, wherein the seconds LEDs are arranged to emit UV light in a wavelength range of 100-380 nm. Hence, the LED filament comprises two linear arrays, wherein each linear array comprises a (respective) plurality of LEDs. The plurality of first LEDs may comprise at least 10 LEDs, preferably at least 20 LEDs, more preferably at least 30 LEDs, most preferably at least 40 LEDs. The plurality of second LEDs may comprise at least 5 LEDs, preferably at least 10 LEDs, more preferably at least 15 LEDs, most preferably at least 20 LEDs. The second linear array is arranged adjacent and parallel to the first linear array. For example, the first and second array may be arranged linearly next to each other and extend along the LED filament. Furthermore, the LED filament comprises a first encapsulant which at least partially encloses the first array of the first LEDs, wherein the first encapsulant comprises a light-scattering material configured to scatter the violet light emitted from the first LEDs. By the term "encapsulant", it is here meant a material, element, arrangement, or the like, which is configured or arranged to at least partially surround, encapsulate and/or enclose the linear array. The light-scattering material is configured to scatter the light. More specifically, the light scattering material may be configured to scatter the violet light in a wavelength range of 380-420 nm. The light-scattering material may enable forward and/or backward scattering. The scattering may be primarily forward scattering or backward scattering. In an embodiment, at least 50% of the violet light emitted from the plurality of first LEDs is scattered by the light scattering material, preferably at least 70%, more preferably at least 80%, most preferably at least 90%. In a further embodiment, at most 10% of the UV light emitted from the plurality of second LEDs is scattered, preferably at most 7%, more preferably at most 5%, most preferably at most 3%.

According to an embodiment of the present invention, the LED filament further comprises a second encapsulant, wherein the second encapsulant is transparent and at least partially encloses the second array of the plurality of second LEDs. By the term "transparent", it is here meant that the second encapsulant comprises a material, composition and/or substance which is transparent and/or translucent. The second encapsulant may have some light scattering properties. For example, at most 10% of the UV light emitted from the plurality of second LEDs is scattered in the second encapsulant, preferably at most 7%, more preferably at most 5%, most preferably at most 3%. The present embodiment is advantageous in that it may improve the disinfection performance of the LED filament, since the UV light may be extracted better from the second LEDs, e.g. via better distribution of the light after it passes through the second encapsulant.

According to an embodiment of the present invention, the light scattering material comprises a silicone matrix with at least one of Al₂O₃, BaSO₄, TiO₂, SiO₂, CaF₂, CaCO₃, and BaTiO₃ particles.

According to an embodiment of the present invention, the first encapsulant is configured to reflect the UV light emitted from the plurality of second LEDs. The present embodiment is advantageous in that it reduces the amount of UV light that may reach the plurality of first LEDs.

According to the present invention, the number of first LEDs, N₁, and the number of second LEDs, N₂, fulfils N₁ ≥ 2·N₂, especially N₁ ≥ 3·N₂, or N₁ ≥ 4·N₂. Furthermore, the number of first LEDs, N₁, per unit length of the first array, L₁, and the number of second LEDs, N₂, per unit length of the second array, L₂, fulfils N₁/L₁ ≥ 2·N₂/L₂, especially fulfil N₁/L₁ ≥ 3·N₂/L₂ or fulfil N₁/L₁ ≥ 4·N₂/L2. In other words, either the number of first LEDs, N₁, is equal to, or more, than four times the number of second LEDs, N₂, or the number of first LEDs per unit length of the first array, N₁/L₁, (i.e. the LED density of the first linear array) is equal to, or more than, two, three or even four times the number of second LEDs per unit length of the second array, N₂/L₂.

The present embodiment is advantageous in that a more efficient and aesthetically pleasing LED light distribution is provided. This is because an improved line emission is achieved for the visible violet light by having a relatively high number of LEDs, while the number of second LEDs emitting non-visible UV light is relatively few in order to provide a more efficient disinfecting lighting, e.g. by generating less heat while still providing sufficient lighting for disinfection.

According to an embodiment of the present invention, the plurality of first LEDs comprises a first subset of LEDs arranged to emit violet light with a first dominant peak wavelength, DPW1, in the range 400-410 nm. By "dominant peak wavelength", it is here meant a centroid wavelength, i.e. a wavelength at which the UV light reaches a maximum intensity. The present embodiment is advantageous in that the LED filament light comprises UV light of a wavelength that is a balanced option between safety and disinfection performance.

According to an embodiment of the present invention, at least one of the plurality of first LEDs comprises a second subset of LEDs arranged to emit violet light with a second dominant peak wavelength, DPW₂, in the range 410-420 nm, and the plurality of first LEDs comprises a third subset of LEDs arranged to emit violet light with a third dominant peak wavelength, DPW₃, in the range 390-400 nm, is fulfilled. The present embodiment is advantageous in that two intervals of the violet spectrum are used in combination, e.g. to provide different ranges of visible light that provide a more aesthetically pleasing effect and/or the level of disinfection performance. For example, the second subset may be arranged on one part of the LED filament, while the third subset is arranged on a different part of the LED filament, such that a user may choose between better disinfection performance and more harmful/less safe light, and limited disinfection performance and less harmful/more safe light by moving within the area being illuminated by the LED filament light. Light in a wavelength range from 380 to 400 nm has a relatively high disinfection performance compared to light in a wavelength range from 400 to 420 nm. Light in a wavelength range from 400 to 420 nm is more visible and is relatively safe compared to light in a wavelength range from 380 to 400 nm. Light in a wavelength range from 400 to 410 nm may provide a balanced/optimal light output, in terms of performance, safety and visibility.

According to an embodiment of the present invention, the encapsulant comprises an elongated shape and has a length of at least 4 cm, and wherein the plurality of first LEDs comprises at least 10 first LEDs, and wherein the distance, d, between adjacent first LEDs of the first array, fulfills d ≤ 4 mm. The present embodiment is advantageous in that the first LEDs may emit violet light in an improved line pattern. As a consequence, the violet light is emitted in a more aesthetically pleasing manner.

According to an embodiment of the present invention, the plurality of second LEDs is arranged to emit UVC light in a wavelength range of 100-280 nm. The present embodiment is advantageous in that the UVC light in the range of 100-280 nm may penetrate the skin less than UV light of a longer wavelength, such as UVB light. Consequently, the UVC light may be less harmful than other types of UV light, for example to the skin of a person. The present embodiment is further advantageous in that the UVA wavelength range has an improved disinfection performance, i.e. an improved operation of inactivating/killing bacteria.

According to an embodiment of the present invention, there is provided a LED device. The LED device comprises at least one LED filament according to any one of the preceding claims, and a carrier, wherein the at least one LED filament is supported by the carrier. It is to be understood that the LED filament may be arranged on the carrier and/or supported by the carrier. By the term "carrier", it is here meant an element, substrate, or the like, arranged to mechanically and/or electrically support LEDs. Hence, the plurality of LEDs may be arranged, mounted and/or mechanically coupled on/to the carrier (e.g. a substrate), wherein the carrier is configured to mechanically and/or electrically support the LEDs. The carrier may furthermore be elongated in order to support the arrays of LEDs of the (elongated) LED filament.

According to an embodiment of the present invention, the LED device further comprises a control unit configured to (individually) control the plurality of first LEDs and the plurality of second LEDs. The LED device further comprises a user interface, wherein the controller is configured to be controlled by an operator via the user interface. The present embodiment is advantageous in that the individual control of the plurality of LEDs as provided by the controller may even further improve the aesthetics and/or light distribution purposes with the advantageous properties of disinfection (bactericidal and/or viricidal) lighting. For example, the controller may hereby be configured to control one or more properties (e.g. intensity) of the LEDs of the first linear array.

According to an embodiment of the present invention, the LED device further comprises a sensor configured to detect at least one of the presence of at least one person and the distance of a person to the sensor. In case the presence of at least one person and/or at least one person is within a predetermined distance, D, to the sensor, is detected by the sensor, the control unit is configured to perform a decrease of the intensity of the UV light emitted by the plurality of second LEDs and/or a switch off of the UV light emitted by the plurality of second LEDs. The sensor may comprise a motion sensor, and/or proximity/presence sensor. The present embodiment is advantageous in that the UV light may be automatically adjusted depending on whether or not a person is detected. As a consequence of this, the LED device may be operated more safely, since the UV light may be decreased and/or turned off in case a person is at risk of being illuminated with UV light. In an embodiment, the switching off of the UV light emitted by the plurality of second LEDs may (only) be based on a (threshold) distance of a person to the sensor. In a further embodiment, the decrease of the intensity of the UV light emitted by the plurality of second LEDs may (only) be based on a presence of at least one person detected by the sensor.

In an embodiment, the LED device comprises a control unit configured to individually control the plurality of first LEDs and the plurality of second LEDs, and a sensor configured to detect at least one of the presence of at least one person and the distance of a person to the sensor. In case at least one of the presence of at least one person, and at least one person is within a predetermined distance, D, to the sensor, is detected by the sensor, the control unit is configured to perform one of a decrease of the intensity of the UV light emitted by the plurality of second LEDs, and a switch off of the UV light emitted by the plurality of second LEDs.

According to an embodiment of the present invention, the control unit is configured to perform a decrease of the intensity of the violet light emitted by the plurality of first LEDs and/or a decrease of the intensity of the violet light emitted by the plurality of first LEDs with a time delay, with respect to the detection of at least one of the presence of at least one person and at least one person being within a predetermined distance, D, to the sensor and/or a switch off of the violet light emitted by the plurality of first LEDs. The decrease of the intensity and/or the switch off of the violet light may be performed with a time delay, with respect to the detection of at least one of the presence of at least one person and at least one person being within a predetermined distance, D, to the sensor. The present embodiment is advantageous in that a time window is created where there is violet light being emitted by the plurality of first LEDs and no UV light being emitted by the plurality of second LEDs, such that a person is less likely to unintentionally be exposed to UV light, which may be harmful. In an embodiment, the time delay may be at least 0.1 second, preferably at least 0.5 second, more preferably at least 0.8 second, most preferably at least 1 second. In a further embodiment, the time delay may be at most 10 second, preferably at most 5 second, more preferably at most 3 second, most preferably at most 2 seconds.

According to an embodiment of the present invention, wherein the LED device comprises a housing configured to at least partially enclose the plurality of first LEDs and the plurality of second LEDs, and wherein the housing is light transmissive for the violet light emitted by the plurality of first LEDs and light transmissive for the UV light emitted by the plurality of second LEDs.

According to an embodiment of the present invention, there is provided a tubular lighting device, comprising a LED device according to an embodiment of the present invention, wherein the housing comprises a first end cap at a first end of the tubular housing, and a second end cap at a second end of the tubular housing, opposite the first end of the tubular housing. The first and second end caps comprise a first pair and second pair of pins, respectively, and a fixture comprising first and second connectors, wherein the first pair and second pair of pins of the tubular housing are configured for mating connection to the first and second connectors, respectively, for mechanical and electrical connection between the tubular housing and the fixture.

Further objectives of, features of, and advantages with, the present invention will become apparent when studying the following detailed disclosure, the drawings and the appended claims. Those skilled in the art will realize that different features of the present invention can be combined to create embodiments other than those described in the following.

### BRIEF DESCRIPTION OF THE DRAWINGS

This and other aspects of the present invention will now be described in more detail, with reference to the appended drawings showing embodiment(s) of the invention.
Figs. 1a-b schematically show LED filaments according to exemplifying embodiments of the present invention,
Fig. 2 schematically shows a LED device according to exemplifying embodiments of the present invention,
Fig. 3 schematically shows a LED device according to exemplifying embodiments of the present invention,
Fig. 4 schematically shows the distribution of the violet light and UV light as a function of time according to exemplifying embodiments of the present invention, and
Fig. 5 schematically shows a tubular LED device according to exemplifying embodiments of the present invention.

### DETAILED DESCRIPTION

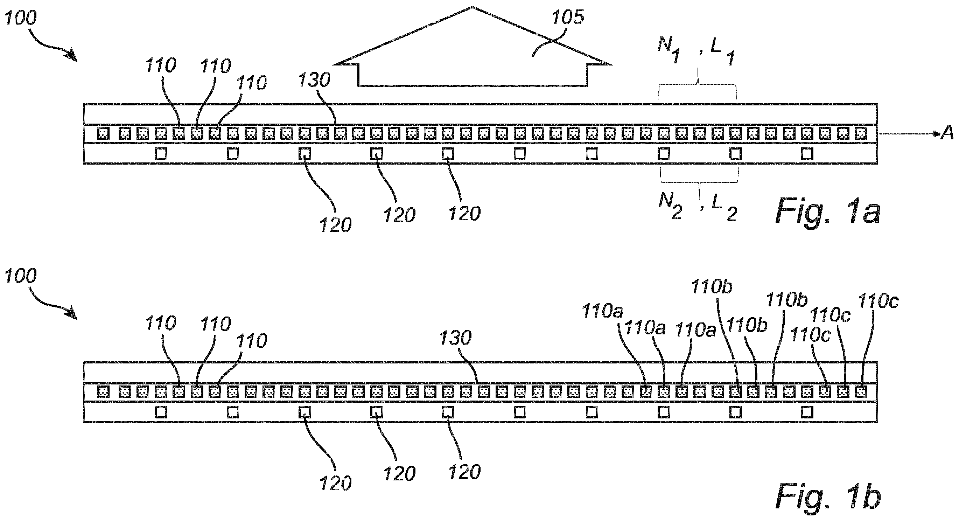

Figs. 1a-b schematically show LED filaments 100 according to exemplifying embodiments of the present invention. Each of the LED filaments 100 comprises a first linear array 110 of a plurality of first light emitting diodes, LEDs. The plurality of first LEDs is arranged to emit violet light in a wavelength range of 380-420 nm. LED filament 100 comprises a second linear array 120 of a plurality of LEDs. The plurality of second LEDs is arranged to emit ultra-violet, UV, light in a wavelength range of 100-380 nm. Alternatively, the plurality of second LEDs may be arranged to emit UVC light in a wavelength range of 100-280 nm. Further, in Figs. 1a-b, the LED filament 100 comprises an elongated shape extending in a first direction, A. The first array 110 and the second array 120 extend linearly in a direction parallel with the first direction, A. The second array 120 is arranged adjacent and parallel to the first array 110. Hence, the LED filament 100 emits filament light 105, wherein the filament light 105 comprises both violet light and UV light. The LED filament light 105 provides both visible violet light and disinfecting UV light. The plurality of first LEDs and the plurality of second LEDs may emit light at the same time, such that the violet light of the plurality of first LEDs may indicate when the UV light of the plurality of second LEDs is emitted. The violet light of the plurality of first LEDS and the UV light of the plurality of second LEDs may be emitted in the same area, at least partially, and/or have, at least partially, the same light distribution cone, such that the violet light may indicate where the UV light is emitted.

In Figs. 1a-b the number of first LEDs, N₁, and the number of second LEDs, N₂, fulfil N₁ ≥ 4·N₂. Furthermore, the number of first LEDs, N₁, per unit length of the first array 110, L₁, and the number of second LEDs, N₂, per unit length of the second array 120, L₂, fulfils N₁/L₁ ≥ 4·N₂/L2. In other words, the number if first LEDs is 4 times higher than the number of second LEDs and the LED density, i.e. the number of LEDs per unit length, is 4 times higher for the first array 110 compared to the second array 120. It is to be understood that, either the number of first LEDs, N₁, may be equal to, or more, than four times the number of second LEDs, N₂, and/or the number of first LEDs per unit length of the first array, N₁/L₁, (i.e. the LED density of the first linear array) is equal to, or more than, four times the number of second LEDs per unit length of the second array, N₂/L₂. Furthermore, it is to be understood that the number of LEDs per unit length for the respective array may vary, and the LED density may also vary. The LED filament 100 comprises a first encapsulant 130, wherein the first encapsulant 130 at least partially encloses the first array 110 of the plurality of first LEDs. It is to be understood that the first encapsulant 130 may enclose some or all of the plurality of first LEDs. Furthermore, it is to be understood that the first encapsulant 130 may fully enclose some, or all, of the plurality of first LEDs. The first encapsulant 130 may comprise a light scattering material. The light scattering material may comprise a silicone matrix with at least one of Al₂O₃, BaSO₄, SiO₂, TiO₂, CaF₂, CaCO₃, and BaTiO₃ particles. Furthermore, the first encapsulant 130 may be configured to reflect UV light emitted from the plurality of second LEDs. For example, the first encapsulant 130 may comprise a material which reflects UV light in the wavelength range of 100-380 nm. The first encapsulant 130 may comprise an elongated shape. The first encapsulant 130 may preferably have a length of at least 4 cm, and the plurality of first LEDs may preferably comprise at least 10 first LEDs, wherein the distance, d, between adjacent first LEDs of the first array 110 preferably fulfils d ≤ 4 mm. It is to be understood that the length of the LED filament 100 and/or the first array 110 and/or the second array 120 may be longer than 4 cm and still achieve a line emission effect for the violet light.

Fig. 1b schematically shows a LED filament 100 according to an exemplifying embodiment of the present invention. The plurality of first LEDs of the first array 110 comprises a first subset 110a of LEDs arranged to emit violet light wit ha first dominant peak wavelength, DPW1, in the range of 400-410 nm. The plurality of first LEDs of the first array 110 further comprises a second subset 110b of LEDs arranged to emit violet light with a second dominant peak wavelength, DPW2, in the range of 410-420 nm. The plurality of first LEDs of the first array 110 further comprises a third subset 110c of LEDs arranged to emit violet light with a third dominant peak wavelength, DPW3, in the range of 390-400 nm. It is to be understood that the plurality of first LEDs may comprise any combination of the first, second and third subset of LEDs.

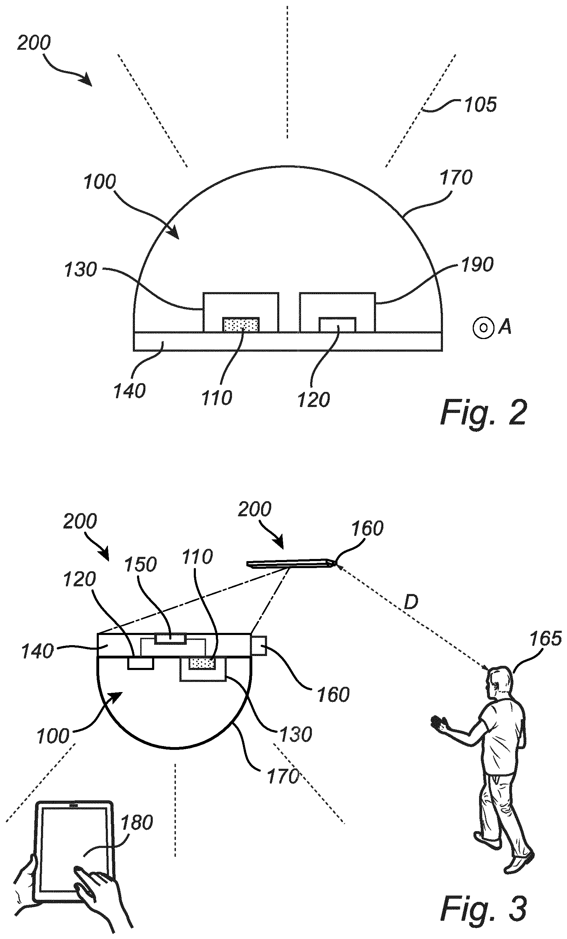

Fig. 2 schematically shows a LED device 200 according to exemplifying embodiments of the present invention. The LED device 200 comprises at least one LED filament 100 configured to emit LED filament light 105. It should be noted that the LED filament 100 is according to an exemplifying embodiment of the present invention and has several features in common with the LED filament 100 shown in Figs. 1a-b, and it is hereby referred to Figs. 1a-b and the associated texts for an increased understanding of at least some of the features and/or functions of the LED filament 100. As indicated by the first direction, A, the at least one LED filament in Fig. 2 is (are) shown perpendicular to the LED filament 100 shown in Fig. 1 for an increased understanding. The LED device 200 comprises a carrier 140. The LED filament 100 is supported/carried by the carrier 140. The LED filament 100 may be arranged on the carrier 140. In fig. 2 the LED filament 100 comprises a first linear array 110 of a plurality of first LEDs, and a second linear array 120 of a plurality of second LEDs. The first linear array 110 and the second linear array 120 are arranged adjacently and in parallel.

The LED filament 100 of the LED device 200 in Fig. 2 comprises a first encapsulant 130 at least partially enclosing the first array 110. The first encapsulant 130 comprises a light scattering material, configured to scatter the violet light from the plurality of first LEDs. The first encapsulant 130 may also be configured to be reflective of the UV light emitted by the plurality of second LEDs. For example, the first encapsulant 130 may comprise a material that reflects the UV light of the plurality of second LEDs. The LED filament 100 further comprises a second encapsulant 190 at least partially enclosing the second array 120. It is to be understood that the second encapsulant 190 may enclose some or all of the plurality of second LEDs. Furthermore, it is to be understood that the second encapsulant 190 may fully enclose some or all of the plurality of second LEDs. The second encapsulant 190 is configured to be transparent. For example, the second encapsulant 190 comprises a transparent material. The second encapsulant 190 may be configured to at least partially direct the UV light, such that the distribution of the UV light may be improved, e.g. to allow for improved disinfection. The LED device 200 comprises a housing 170 configured to at least partially enclose the plurality of first LEDs and the plurality of second LEDs. The housing 170 is light transmissive for the violet light emitted by the plurality of first LEDs and light transmissive for the UV light emitted by the plurality of second LEDs. The housing 170 may comprise an elongated half-cylinder shape extending along the LED filament 100. The housing 170 may be attached/mounted/fixated to the carrier 140. The housing 170 may at least partially enclose the LED filament 100 between the carrier 140 and the housing 170.

The LED filament light 105 comprises a combination of light of the first plurality of LEDs and the second plurality of LEDs. One part of the LED filament light 105 is visible light in the wavelength range of 380-420 nm, and a second part is non-visible UV light in the wavelength range of 100-380 nm. The combination of the violet light and the UV light provides a visible line emission of violet light together with, and/or close to, UV light which may disinfect. The visible violet light of the plurality of first LEDs may act as a visual guide to see what is illuminated by the UV light. The violet light of the plurality of first LEDs may, at least partially, be a disinfecting light.

Fig. 3 schematically shows a LED device 200 according to an exemplifying embodiment of the present invention. It should be noted that the LED device 200 shown in Fig. 3 has several features in common with the LED device 200 shown in Fig. 2, and it is hereby referred to Fig. 2 and the associated text for an increased understanding of some of the features and/or functions of the LED device 200. The LED device 200 comprises a LED filament 100. The LED filament 100 comprises a first array 110 and a second array 120 of LEDs, and a first encapsulant 130 at least partially enclosing the first array 110. The first array 110 and the second array 120 are supported/arranged on the carrier 140. The LED device 200 comprises a housing 170 at least partially enclosing the first array 110 and the second array 120.

In Fig. 3, the LED device further comprises a control unit 150 configured to individually control the plurality of first LEDs and the plurality of second LEDs. The LED device 200 further comprises a user interface 180, wherein the control unit 150 is configured to be controlled by an operator via the user interface 180. The control unit 150 may form part of the LED device and/or may be arranged external to the LED device. The LED device 200 further comprises a (schematically indicated) sensor 160. The sensor 160 may form part of the LED device and/or may be arranged external to the LED device. The sensor 160 is configured to detect a presence of at least one person and/or a distance of a person 165 to the sensor 160. In case the presence of the person(s) 165 and/or the person(s) 165 is within a predetermined distance, D, to the sensor 160, is detected by the sensor 160, the control unit 150 is configured to decrease the intensity of the UV light emitted by the plurality of second LEDs and/or switch off the UV light emitted by the plurality of second LEDs. The control unit 150 is further configured to decrease the intensity of the violet light emitted by the plurality of first LEDs and/or switch off of the violet light emitted by the plurality of first LEDs. The decrease of intensity and/or the switch off may be performed with a time delay, with respect to the detection of the presence of the person(s) 165 and/or the person(s) 165 being within a predetermined distance, D, to the sensor 160.

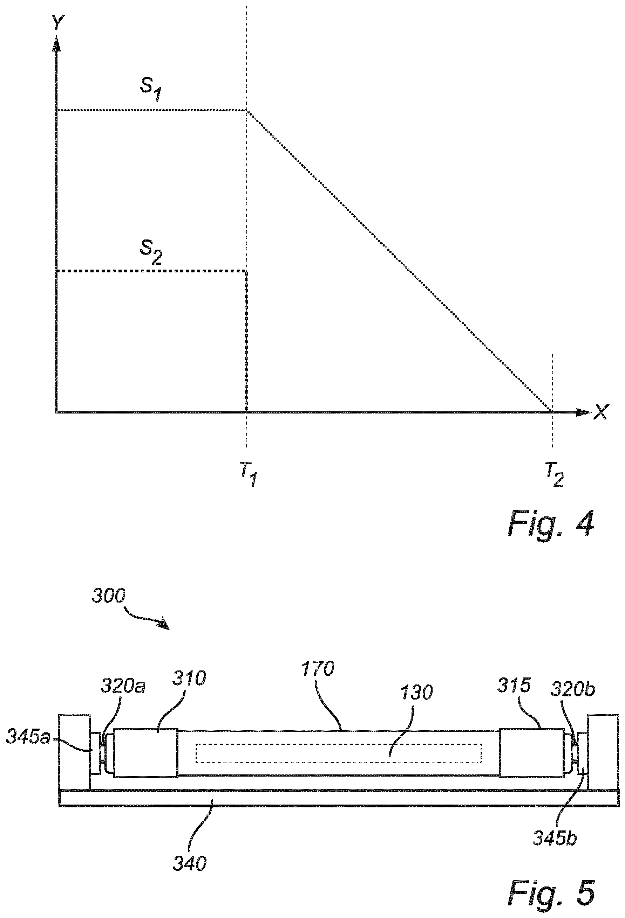

Fig. 4 schematically shows the distribution of the violet light, represented by a first series, S₁, provided by the plurality of first LEDs and the UV light, represented by a second series, S₂, provided by the plurality of second LEDs with a respective intensity (y-axis, arb. units) as a function of time (x-axis, arb. units). In Fig. 4, the violet light, S₁, is gradually decreased after a time, T₁, wherein T₁ is the time when a person is detected by a sensor and/or when at least one person is within a predetermined distance, D, of the sensor. A control unit may be configured to control the intensity of the plurality of first LEDs such that the intensity is decreased over time. An example of the sensor and the control unit used is depicted and described in Fig. 3 and the related text. The violet light, S₁, is decreased until a time, T₂, when the plurality of first LEDs is shut off and/or the intensity of the violet light reaches zero, or a relatively low value. The decrease of the violet light, S₁, may be linear and/or non-linear. The UV light, S₂, may be turned off directly/promptly/instantaneously after the time, T₁. The control unit may be configured to control the plurality of second LEDs such that they are turned off. It is to be understood that the time T₂ occurs after the time T₁. Consequently, the plurality of second LEDs is shut off before the plurality of first LEDs. The delay, T₂-T₁, between the shut off time of the plurality of second LEDs and the plurality of first LEDs may be in the interval 0.1 to 6 seconds.

Fig. 5 schematically shows a tubular LED device, TLED, 300 according to exemplifying embodiments of the present invention. The TLED 300 comprises a housing 170. The housing 170 encloses, at least partially, a LED filament (not indicated) according to an embodiment of the present invention. Consequently, the housing 170 encloses, at least partially, the plurality of first LEDs and the plurality of second LEDs. It is to be understood that the housing 170 may fully enclose the plurality of first LEDs and plurality of second LEDs, and/or the whole LED filament 100. The housing 170 is light transmissive, both for the violet light transmitted by the plurality of first LEDs and for the UV light emitted by the plurality of second LEDs. The housing 170 comprises a first end cap 310 at a first end of the housing 170. The housing 170 is a tubular housing which elongates in the length direction of the TLED. The tubular housing 170 comprises a second end cap 315 at a second end of the tubular housing 170, opposite the first end of the tubular housing 170. The first and second end caps 310, 315 comprise a first pair and second pair of fins 320a, 320b, respectively. The TLED 300 further comprises a fixture 340. The fixture 340 comprises first and second connectors 345a, 345b. The first pair and second pair of pins 320a, 320b are configured for mating connection to the first and second connectors 345a, 345b, respectively, for mechanical and electrical connection between the tubular housing 170 and the fixture 140.

## Claims

1. A light emitting diode, LED, filament (100), configured to emit LED filament light (105), comprising
a first linear array (110) of a plurality of first light emitting diodes, LEDs, wherein the plurality of first LEDs is arranged to emit violet light in a wavelength range of 380-420 nm,
a second linear array (120) of a plurality of second LEDs, wherein the plurality of second LEDs is arranged to emit ultra-violet, UV, light in a wavelength range of 100-380 nm, and wherein the second array is arranged adjacent and parallel to the first array,
a first encapsulant (130) at least partially enclosing the first array of the plurality of first LEDs, wherein the first encapsulant comprises a light-scattering material configured to scatter the violet light emitted from the plurality of first LEDs, and
wherein the number of first LEDs, N₁, and the number of second LEDs, N₂, fulfil N₁ ≥ 2·N₂, especially N₁ ≥ 3·N₂, or N₁ ≥ 4·N₂, and the number of first LEDs, N₁, per unit length of the first array, L₁, and the number of second LEDs, N₂, per unit length of the second array, L₂, fulfil N₁/L₁ ≥ 2·N₂/L₂, especially N₁/L₁ ≥ 3·N₂/L₂ or N₁/L₁ ≥ 4·N₂/L2.

2. The LED filament according to claim 1, further comprising a second encapsulant (190), wherein the second encapsulant is transparent and at least partially encloses the second array of the plurality of second LEDs.

3. The LED filament according to claim 1 or 2, wherein the light scattering material comprises a silicone matrix with at least one of Al₂O₃, BaSO₄, TiO₂, SiO₂, CaF₂, CaCO₃, and BaTiO₃ particles.

4. The LED filament according to any one of the preceding claims, wherein the first encapsulant is configured to reflect at least part of the UV light emitted from the plurality of second LEDs.

5. The LED filament according to any one of the preceding claims, wherein the plurality of first LEDs comprises a first subset (110a) of LEDs arranged to emit violet light with a first dominant peak wavelength, DPW₁, in the range 400-410 nm.

6. The LED filament according to any one of the preceding claims, wherein at least one of
the plurality of first LEDs comprises a second subset (110b) of LEDs arranged to emit violet light with a second dominant peak wavelength, DPW₂, in the range 410-420 nm, and
the plurality of first LEDs comprises a third subset (110c) of LEDs arranged to emit violet light with a third dominant peak wavelength, DPW₃, in the range 380-400 nm,
is fulfilled.

7. The LED filament according to any one of the preceding claims, wherein the encapsulant comprises an elongated shape and has a length of at least 4 cm, and wherein the plurality of first LEDs comprises at least 10 first LEDs, and wherein the distance, d, between adjacent first LEDs of the first array, fulfills d ≤ 4 mm.

8. The LED filament according to any one of the preceding claims, wherein the plurality of second LEDs is arranged to emit UVC light in a wavelength range of 100-280 nm.

9. A light emitting diode, LED, device (200), comprising
at least one LED filament according to any one of the preceding claims, and
a carrier (140), wherein the at least one LED filament is supported by the carrier.

10. The LED device according to claim 9, further comprising
a control unit (150) configured to individually control the plurality of first LEDs and the plurality of second LEDs, and
a user interface (180), wherein the control unit is configured to be controlled by an operator via the user interface.

11. The LED device according to claim 10, further comprising
a sensor (160) configured to detect at least one of the presence of at least one person (165) and the distance of a person to the sensor, and
in case at least one of
the presence of at least one person, and
at least one person is within a predetermined distance, D, to the sensor,
is detected by the sensor,
the control unit is configured to perform one of
a decrease of the intensity of the UV light emitted by the plurality of second LEDs, and
a switch off of the UV light emitted by the plurality of second LEDs.

12. The LED device according to claim 11, wherein the control unit is configured to perform one of
a decrease of the intensity of the violet light emitted by the plurality of first LEDs,
a decrease of the intensity of the violet light emitted by the plurality of first LEDs with a time delay, with respect to the detection of at least one of the presence of at least one person and at least one person being within a predetermined distance, D, to the sensor, and
a switch off of the violet light emitted by the plurality of first LEDs with a time delay, with respect to the detection of at least one of the presence of at least one person and at least one person being within a predetermined distance, D, to the sensor.

13. The LED device according to any one of claims 9 to 12, wherein the LED device comprises a housing (170) configured to at least partially enclose the plurality of first LEDs and the plurality of second LEDs, and wherein the housing is light transmissive for the violet light emitted by the plurality of first LEDs and light transmissive for the UV light emitted by the plurality of second LEDs.

14. A tubular lighting device (300), comprising
the LED device according to claim 13,
wherein the housing comprises
a first end cap (310) at a first end of the tubular housing, and
a second end cap (315) at a second end of the tubular housing, opposite the first end of the tubular housing,
wherein the first and second end caps comprise a first pair and second pair of pins (320a, 320b), respectively,
a fixture (340) comprising first and second connectors (345a, 345b), wherein the first pair and second pair of pins of the tubular housing are configured for mating connection to the first and second connectors, respectively, for mechanical and electrical connection between the tubular housing and the fixture.

## Patentansprüche

1. Leuchtdiodenfilament, LED-Filament, (100), das konfiguriert ist, um LED-Filamentlicht (105) zu emittieren, umfassend
eine erste lineare Anordnung (110) von einer Vielzahl von ersten Leuchtdioden, LEDs, wobei die Vielzahl von ersten LEDs eingerichtet ist, um violettes Licht in einem Wellenlängenbereich von 380-420 nm zu emittieren,
eine zweite lineare Anordnung (120) von einer Vielzahl von zweiten LEDs, wobei die Vielzahl von zweiten LEDs eingerichtet ist, um ultraviolettes Licht, UV-Licht, in einem Wellenlängenbereich von 100-380 nm zu emittieren, und wobei die zweite Anordnung angrenzend an und parallel zu der ersten Anordnung eingerichtet ist,
ein erstes Verkapselungsmittel (130), das die erste Anordnung der Vielzahl von ersten LEDs mindestens teilweise umschließt, wobei das erste Verkapselungsmittel ein lichtstreuendes Material umfasst, das konfiguriert ist, um das violette Licht zu streuen, das von der Vielzahl von ersten LEDs emittiert wird, und
wobei die Anzahl von ersten LEDs, N₁, und die Anzahl von zweiten LEDs, N₂, N₁ ≥ 2·N₂, insbesondere N₁ ≥ 3·N₂ oder N₁ ≥ 4·N₂ erfüllen und die Anzahl von ersten LEDs, N₁, pro Einheitslänge der ersten Anordnung, L₁, und die Anzahl von zweiten LEDs, N₂, pro Einheitslänge der zweiten Anordnung, L₂, N₁/L₁ ≥ 2·N₂/L₂, insbesondere N₁/L₁ ≥ 3·N₂/L₂ oder N₁/L₁ ≥ 4·N₂/L₂ erfüllen.

2. LED-Filament nach Anspruch 1, ferner umfassend ein zweites Verkapselungsmittel (190), wobei das zweite Verkapselungsmittel transparent ist und die zweite Anordnung der Vielzahl von zweiten LEDs mindestens teilweise umschließt.

3. LED-Filament nach Anspruch 1 oder 2, wobei das lichtstreuende Material eine Silikonmatrix mit mindestens einem von Al₂O₃-, BaSO₄-, TiO₂-, SiO₂-, CaF₂-, CaCO₃- und BaTiO₃-Teilchen umfasst.

4. LED-Filament nach einem der vorstehenden Ansprüche, wobei das erste Verkapselungsmittel konfiguriert ist, um mindestens einen Teil des UV-Lichts zu reflektieren, das von der Vielzahl von zweiten LEDs emittiert wird.

5. LED-Filament nach einem der vorstehenden Ansprüche, wobei die Vielzahl von ersten LEDs einen ersten Teilsatz (110a) von LEDs umfasst, die eingerichtet sind, um violettes Licht mit einer ersten farbtongleichen Spitzenwellenlänge, DPW₁, in dem Bereich von 400-410 nm zu emittieren.

6. LED-Filament nach einem der vorstehenden Ansprüche, wobei mindestens eines von
die Vielzahl von ersten LEDs umfasst einen zweiten Teilsatz (110b) von LEDs, die eingerichtet sind, um violettes Licht mit einer zweiten farbtongleichen Spitzenwellenlänge, DPW₂, in dem Bereich von 410-420 nm zu emittieren, und
die Vielzahl von ersten LEDs umfasst einen dritten Teilsatz (110c) von LEDs, die eingerichtet sind, um violettes Licht mit einer dritten farbtongleichen Spitzenwellenlänge, DPW₃, in dem Bereich von 380-400 nm zu emittieren,
erfüllt ist.

7. LED-Filament nach einem der vorstehenden Ansprüche, wobei das Verkapselungsmittel eine längliche Form umfasst und eine Länge von mindestens 4 cm aufweist und wobei die Vielzahl von ersten LEDs mindestens 10 erste LEDs umfasst und wobei die Entfernung, d, zwischen angrenzenden ersten LEDs der ersten Anordnung d ≤ 4 mm erfüllt.

8. LED-Filament nach einem der vorstehenden Ansprüche, wobei die Vielzahl von zweiten LEDs eingerichtet ist, um UVC-Licht in einem Wellenlängenbereich von 100-280 nm zu emittieren.

9. Leuchtdiodenvorrichtung, LED-Vorrichtung, (200), umfassend
mindestens ein LED-Filament nach einem der vorstehenden Ansprüche und
einen Träger (140), wobei das mindestens eine LED-Filament durch den Träger gestützt wird.

10. LED-Vorrichtung nach Anspruch 9, ferner umfassend
eine Steuereinheit (150), die konfiguriert ist, um die Vielzahl von ersten LEDs und die Vielzahl von zweiten LEDs individuell zu steuern, und
eine Benutzerschnittstelle (180), wobei die Steuereinheit konfiguriert ist, um durch einen Betreiber über die Benutzerschnittstelle gesteuert zu werden.

11. LED-Vorrichtung nach Anspruch 10, ferner umfassend
einen Sensor (160), der konfiguriert ist, um mindestens eines von der Anwesenheit mindestens einer Person (165) und der Entfernung einer Person zu dem Sensor zu erfassen, und wobei,
in einem Fall, dass mindestens eines von
der Anwesenheit mindestens einer Person und
mindestens eine Person, die sich innerhalb einer vorbestimmten Entfernung, D, zu dem Sensor befindet,
durch den Sensor erfasst wird,
die Steuereinheit konfiguriert ist, um eines durchzuführen von
einer Verringerung der Intensität des UV-Lichts, das durch die Vielzahl von zweiten LEDs emittiert wird, und
einem Ausschalten des UV-Lichts, das durch die Vielzahl von zweiten LEDs emittiert wird.

12. LED-Vorrichtung nach Anspruch 11, wobei die Steuereinheit konfiguriert ist, um eines durchzuführen von
einer Verringerung der Intensität des violetten Lichts, das durch die Vielzahl von ersten LEDs emittiert wird,
einer Verringerung der Intensität des violetten Lichts, das durch die Vielzahl von ersten LEDs emittiert wird, mit einer Zeitverzögerung in Bezug auf die Erfassung von mindestens einem von der Anwesenheit mindestens einer Person und mindestens einer Person, die sich innerhalb einer vorbestimmten Entfernung, D, zu dem Sensor befindet, und
einem Ausschalten des violetten Lichts, das durch die Vielzahl von ersten LEDs emittiert wird, mit einer Zeitverzögerung in Bezug auf die Erfassung von mindestens einem von der Anwesenheit mindestens einer Person und mindestens einer Person, die sich innerhalb einer vorbestimmten Entfernung, D, zu dem Sensor befindet.

13. LED-Vorrichtung nach einem der Ansprüche 9 bis 12, wobei die LED-Vorrichtung ein Gehäuse (170) umfasst, das konfiguriert ist, um die Vielzahl von ersten LEDs und die Vielzahl von zweiten LEDs mindestens teilweise zu umschließen, und wobei das Gehäuse lichtdurchlässig für das ultraviolette Licht ist, das durch die Vielzahl von ersten LEDs emittiert wird, und lichtdurchlässig für das UV-Licht ist, das durch die Vielzahl von zweiten LEDs emittiert wird.

14. Rohrförmige Beleuchtungsvorrichtung (300), umfassend
die LED-Vorrichtung nach Anspruch 13,
wobei das Gehäuse umfasst
eine erste Endkappe (310) an einem ersten Ende des rohrförmigen Gehäuses und
eine zweite Endkappe (315) an einem zweiten Ende des rohrförmigen Gehäuses, gegenüber dem ersten Ende des rohrförmigen Gehäuses,
wobei die erste und die zweite Endkappe ein erstes Paar beziehungsweise ein zweites Paar von Stiften (320a, 320b) umfassen,
eine Halterung (340), umfassend einen ersten und einen zweiten Verbinder (345a, 345b), wobei das erste Paar und das zweite Paar von Stiften des rohrförmigen Gehäuses für eine passende Verbindung mit dem ersten beziehungsweise dem zweiten Verbinder konfiguriert sind, für eine mechanische und elektrische Verbindung zwischen dem rohrförmigen Gehäuse und der Halterung.

## Revendications

1. Filament à diode électroluminescente, DEL, (100) configuré pour émettre une lumière de filament à DEL (105), comprenant
un premier réseau linéaire (110) d'une pluralité de premières diodes électroluminescentes, DEL, dans lequel la pluralité de premières DEL est agencée pour émettre une lumière violette dans une plage de longueur d'onde de 380 à 420 nm,
un second réseau linéaire (120) d'une pluralité de secondes DEL, dans lequel la pluralité de secondes DEL est agencée pour émettre une lumière ultraviolette, UV, dans une plage de longueurs d'onde de 100 à 380 nm, et dans lequel le second réseau est agencé à proximité du premier réseau et parallèle à celui-ci,
un premier agent d'encapsulation (130) renfermant au moins partiellement le premier réseau de la pluralité de premières DEL, dans lequel le premier agent d'encapsulation comprend un matériau de diffusion de lumière configuré pour diffuser la lumière violette émise à partir de la pluralité de premières DEL, et
dans lequel le nombre de premières DEL, N₁, et le nombre de secondes DEL, N₂, satisfont à N₁ ≥ 2 · N₂, en particulier N₁ ≥ 3 · N₂, ou N₁ ≥ 4 · N₂, et le nombre de premières DEL, N₁, par unité de longueur du premier réseau, L₁, et le nombre de secondes DEL, N₂, par unité de longueur du second réseau, L₂, satisfont à N₁/L₁ ≥ 2 · N₂/L₂, en particulier N₁/L₁ ≥ 3 · N₂/L₂ ou N₁/L₁ ≥ 4 · N₂/L₂.

2. Filament à DEL selon la revendication 1, comprenant en outre un second agent d'encapsulation (190), dans lequel le second agent d'encapsulation est transparent et renferme au moins partiellement le second réseau de la pluralité de secondes DEL.

3. Filament à DEL selon la revendication 1 ou 2, dans lequel le matériau de diffusion de lumière comprend une matrice de silicone avec au moins l'une parmi les particules Al₂O₃, BaSO₄, TiO₂, SiO₂, CaF₂, CaCO₃ et BaTiO₃.

4. Filament à DEL selon l'une quelconque des revendications précédentes, dans lequel le premier agent d'encapsulation est configuré pour réfléchir au moins une partie de la lumière UV émise à partir de la pluralité de secondes DEL.

5. Filament à DEL selon l'une quelconque des revendications précédentes, dans lequel la pluralité de premières DEL comprend un premier sous-ensemble (110a) de DEL agencées pour émettre une lumière violette avec une première longueur d'onde de crête dominante, DPW₁, dans la plage de 400 à 410 nm.

6. Filament à DEL selon l'une quelconque des revendications précédentes, dans lequel au moins l'une parmi
la pluralité de premières DEL comprend un second sous-ensemble (110b) de DEL agencées pour émettre une lumière violette avec une deuxième longueur d'onde de crête dominante, DPW₂, dans la plage de 410 à 420 nm, et
la pluralité de premières DEL comprend un troisième sous-ensemble (110c) de DEL agencées pour émettre une lumière violette avec une troisième longueur d'onde de crête dominante, DPW₃, dans la plage de 380 à 400 nm,
est satisfaite.

7. Filament à DEL selon l'une quelconque des revendications précédentes, dans lequel l'agent d'encapsulation comprend une forme allongée et a une longueur d'au moins 4 cm, et dans lequel la pluralité de premières DEL comprend au moins 10 premières DEL, et dans lequel la distance, d, entre des premières DEL adjacentes du premier réseau, satisfait à d ≤ 4 mm.

8. Filament à DEL selon l'une quelconque des revendications précédentes, dans lequel la pluralité de secondes DEL est agencée pour émettre une lumière UVC dans une plage de longueurs d'onde de 100 à 280 nm.

9. Dispositif à diode électroluminescente, DEL (200), comprenant
au moins un filament à DEL selon l'une quelconque des revendications précédentes, et
un support (140), dans lequel l'au moins un filament à DEL est supporté par le support.

10. Dispositif à DEL selon la revendication 9, comprenant en outre
une unité de commande (150) configurée pour commander individuellement la pluralité de premières DEL et la pluralité de secondes DEL, et
une interface utilisateur (180), dans lequel l'unité de commande est configurée pour être commandée par un opérateur par l'intermédiaire de l'interface utilisateur.

11. Dispositif à DEL selon la revendication 10, comprenant en outre
un capteur (160) configuré pour détecter au moins l'une parmi la présence d'au moins une personne (165) et la distance d'une personne par rapport au capteur, et
dans le cas où au moins l'une parmi
la présence d'au moins une personne, et
au moins une personne se trouve à une distance prédéterminée, D, du capteur,
est détectée par le capteur,
l'unité de commande est configurée pour réaliser l'une parmi
une diminution de l'intensité de la lumière UV émise par la pluralité de secondes DEL, et
une extinction de la lumière UV émise par la pluralité de secondes DEL.

12. Dispositif à DEL selon la revendication 11, dans lequel l'unité de commande est configurée pour réaliser l'une parmi
une diminution de l'intensité de la lumière violette émise par la pluralité de premières DEL,
une diminution de l'intensité de la lumière violette émise par la pluralité de premières DEL avec un décalage, par rapport à la détection d'au moins l'une parmi la présence d'au moins une personne et d'au moins une personne se trouvant à une distance prédéterminée, D, du capteur, et
une extinction de la lumière violette émise par la pluralité de premières DEL avec un décalage, par rapport à la détection d'au moins l'une parmi la présence d'au moins une personne et d'au moins une personne se trouvant à une distance prédéterminée, D, du capteur.

13. Dispositif à DEL selon l'une quelconque des revendications 9 à 12, dans lequel le dispositif à DEL comprend un boîtier (170) conçu pour renfermer au moins partiellement la pluralité de premières DEL et la pluralité de secondes DEL, et dans lequel le boîtier est transmissif à la lumière pour la lumière violette émise par la pluralité de premières DEL et transmissif à la lumière pour la lumière UV émise par la pluralité de secondes DEL.

14. Dispositif d'éclairage tubulaire (300), comprenant
le dispositif à DEL selon la revendication 13,
dans lequel le boîtier comprend
un premier capuchon d'extrémité (310) au niveau d'une première extrémité du boîtier tubulaire, et
un second capuchon d'extrémité (315) au niveau d'une seconde extrémité du boîtier tubulaire, opposée à la première extrémité du boîtier tubulaire,
dans lequel les premier et second capuchons d'extrémité comprennent une première paire et une seconde paire de broches (320a, 320b), respectivement,
une fixation (340) comprenant de premier et second connecteurs (345a, 345b), dans lequel la première paire et la seconde paire de broches du boîtier tubulaire sont conçues pour une connexion par accouplement avec les premier et second connecteurs, respectivement, pour une liaison mécanique et une connexion électrique entre le boîtier tubulaire et la fixation.
